(19) Europäisches Patentamt / European Patent Office / Office européen des brevets

(11) **EP 2 527 821 B1**

(12) **EUROPÄISCHE PATENTSCHRIFT**

(45) Veröffentlichungstag und Bekanntmachung des Hinweises auf die Patenterteilung:
**10.06.2015   Patentblatt 2015/24**

(51) Int Cl.:
**G01N 22/00** *(2006.01)*     **G01N 33/22** *(2006.01)*

(21) Anmeldenummer: **12002866.7**

(22) Anmeldetag: **24.04.2012**

(54) **Vorrichtung zur Bestimmung des Volumenanteils wenigstens einer Komponente eines mehrphasigen Mediums**

Device for determining the volume fraction of at least one component of a multi-phase medium

Dispositif de détermination de la fraction volumique d'au moins un composant d'un milieu multiphasique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.05.2011   DE 102011102991**

(43) Veröffentlichungstag der Anmeldung:
**28.11.2012   Patentblatt 2012/48**

(73) Patentinhaber: **KROHNE Messtechnik GmbH 47058 Duisburg (DE)**

(72) Erfinder: **Baer, Christoph 45525 Hattingen (DE)**

(74) Vertreter: **Gesthuysen Patent- und Rechtsanwälte Patentanwälte Postfach 10 13 54 45013 Essen (DE)**

(56) Entgegenhaltungen:
EP-B1- 1 899 690     DE-B3-102004 057 087
JP-A- 2002 350 364     US-A- 5 619 143

- BAER C ET AL: "Conceptual design of a procedure for density monitoring of pulverized fuels in pneumatic conveying systems with microwaves (8â 12 GHz)", MICROWAVE CONFERENCE (GEMIC), 2011 GERMAN, IEEE, 14. März 2011 (2011-03-14), Seiten 1-4, XP031863184, ISBN: 978-1-4244-9225-1

**Beschreibung**

Die Erfindung betrifft eine Vorrichtung zur Bestimmung des Volumenanteils wenigstens einer Komponente eines mehrphasigen Mediums auf Grundlage der Laufzeit eines in das mehrphasige Medium emittierten elektromagnetischen Nutzsignals, mit wenigstens einer Sendevorrichtung zur Emittierung des Nutzsignals in das mehrphasige Medium, mit wenigstens einer Empfangsvorrichtung zum Empfang des Nutzsignals und mit einer Auswertevorrichtung zur Bestimmung der Laufzeit des Nutzsignals zwischen der Sendevorrichtung und der Empfangsvorrichtung. Darüber hinaus betrifft die Erfindung auch ein Verfahren zum Betreiben einer solchen Vorrichtung zur Bestimmung des Volumenanteils wenigstens einer Komponente eines mehrphasigen Mediums.

Der Volumenanteil einer Komponente eines aus mehreren Komponenten bestehenden mehrphasigen Mediums kann in technischen Prozessen aus ganz verschiedenen Gründen von Interesse sein. Beispielsweise bei der Förderung von mehrphasigen Medien interessiert im Regelfall die Zusammensetzung und damit die Dichte des geförderten Mediums, da nur so eine Aussage darüber getroffen werden kann, wie der momentane Fördergrad innerhalb eines Prozesses ist oder welche Mengen innerhalb einer Zeiteinheit in welcher Zusammensetzung gefördert worden sind. Beispielhaft ist hier zu erwähnen die pneumatische Förderung, die eine spezielle Art der Schüttgutförderung ist, und bei der Gase - beispielsweise Luft oder Stickstoff - als förderndes Medium verwendet werden. Als gefördertes Medium hingegen wird das zu transportierende Schüttgut bezeichnet. Üblicherweise erfolgt die Förderung durch Rohre oder Schläuche, die gegen den Außenraum abgedichtet sind. Eine spezielle Anwendung ist z. B. die Förderung von gemahlener Kohle, die zur Beschickung von Brennkammern von Kohlekraftwerken oder Hochöfen verwendet wird.

Eine wichtige Prozessgröße bei der Förderung mehrphasiger Medien ist der Massefluss des geförderten Mediums, wobei gilt:

$$\dot{m} = \frac{\partial m}{\partial t} = \rho \cdot \dot{V}$$

Der Volumenstrom V des geförderten Mediums wird wiederum beschrieben durch:

$$\dot{V} = \frac{\partial V}{\partial t} = v \cdot A \cdot \zeta,$$

wobei v die Geschwindigkeit des geförderten Mediums darstellt, A die Querschnittsfläche des Förderrohres und ζ der Volumenanteil des geförderten Mediums ist.

Unabhängig von dem dargestellten Beispiel eines pneumatisch geförderten Schüttgutes ist aus dem Stand der Technik bekannt, auf den Volumenanteil eines mehrphasigen Mediums - beispielsweise eines zweiphasigen Strömungsgemisches - zu schließen durch die messtechnische Erfassung der Ausbreitungsgeschwindigkeit von elektromagnetischen Wellen in dem Medium, da diese Ausbreitungsgeschwindigkeit bekanntermaßen materialabhängig ist, nämlich abhängt von der relativen Permittivität $\varepsilon_r$ und der relativen Permeabilität $\mu_r$ eines Medium:

$$c = \frac{1}{\sqrt{\varepsilon_0 \cdot \varepsilon_r \cdot \mu_0 \cdot \mu_r}} = \frac{c_0}{\sqrt{\varepsilon_r \cdot \mu_r}}$$

Wenn die magnetischen Eigenschaften der einzelnen Medien des mehrphasigen Mediums vernachlässigbar sind, ergibt sich die Ausbreitungsgeschwindigkeit elektromagnetischer Wellen näherungsweise zu $c_0/\sqrt{\varepsilon_r}$.

Es ist bekannt, dass sich die effektive relative Permittivität eines mehrphasigen Mediums, insbesondere eines Gas-Feststoffgemisches, mittels geeigneter Mischungsformeln bestimmen lässt, wobei Bestandteile jeder Mischungsformel die relativen Permittivitäten der einzelnen gemischten Medien sowie deren Volumenanteil am Gesamtvolumen sind. Wenn die Ausbreitungsgeschwindigkeit von elektromagnetischen Wellen innerhalb des mehrphasigen Mediums messtechnisch bestimmt wird, kann über eine vorgenannte Mischungsformel auf den Volumenanteil des geförderten Mediums geschlossen werden, worauf vorliegend im Einzelnen nicht eingegangen werden soll, Einzelheiten finden sich in der Fachliteratur (z. B. Sihvola, A: "Mixing Rules with complex Dielectric Coefficients", Subsurface Sensing Technologies and Applications, Vol. 1, No. 4, 2000).

Aus dem Stand der Technik ist ferner bekannt, dass zur Bestimmung des Volumenanteils der Komponenten eines zweiphasigen Mediums zunächst eine Leermessung der Ausbreitungsgeschwindigkeit elektromagnetischer Wellen vorgenommen wird, bei der der Innenraum des Förderrohres mit einem Medium bekannter Permittivität gefüllt ist. Über die Änderung der Ausbreitungsgeschwindigkeit im Vergleich zur Leermessung lässt sich dann eine Aussage über die effektive relative Permittivität des mehrphasigen Mediums treffen, das beispielsweise in Form einer Zweiphasenströmung vorliegen kann. Mit dieser Information lässt sich dann der interessierende Volumenanteil des geförderten Mediums berechnen. Es ist bekannt, eine solche Bestimmung mittels einer Transmissionsmessung vorzunehmen (Baer, C., Musch, T., Gerding, M.: "Conceptual Design of a Procedure for Density Monitoring of Pulverized Fuels in Pneumatic Conveying Systems with Microwaves (8-12 GHz)", Proceedings of the 6th German Microwave Conference, March 14-16, 2011, Darmstadt, Germany).

Hierbei wird das das mehrphasige Medium enthaltende Volumen mit einem von der Sendevorrichtung emittierten Nutzsignal beaufschlagt, wobei das Nutzsignal das mehrphasige Medium durchläuft und schließlich mit einer Empfangsvorrichtung wieder aufgenommen wird. Bei bekannter Länge des Laufweges zwischen der Sendevorrichtung und der Empfangsvorrichtung und der Messung der Laufzeit des Nutzsignals kann auf die Ausbreitungsgeschwindigkeit der in Form des Nutzsignals vorliegenden elektromagnetischen Welle geschlossen werden und daraus letztendlich auch den Volumenanteil der interessierenden Komponente des mehrphasigen Mediums. Mit einem Netzwerkanalysator können in einem labormäßigen Aufbau interessierende Informationen über das Übertragungsverhalten des von dem mehrphasigen Medium erfüllten Raumes erhalten werden, jedoch ist diese Lösung gerätetechnisch aufwendig und demzufolge auch kostspielig. Darüber hinaus existiert hier das Problem, dass das von der Sendevorrichtung in das mehrphasige Medium emittierte Nutzsignal nicht nur auf direktem Wege durch das Medium zu der Empfangsvorrichtung gelangt, sondern vielmehr auch Störsignale empfangen werden, die beispielsweise resultieren aus Reflektionen an den Wandungen eines das mehrphasige Medium beherbergenden Behältnisses oder eines Rohres, so dass das Erkennen des eigentlich interessierenden Nutzsignals innerhalb des empfangenen Gesamtsignals Schwierigkeiten bereitet.

Aufgabe der vorliegenden Erfindung ist es, eine Vorrichtung zur Bestimmung des Volumenanteils wenigstens einer Komponente eines mehrphasigen Mediums und ein diesbezügliches Verfahren zum Betreiben einer solchen Vorrichtung anzugeben, die die Bestimmung der Laufzeit des Nutzsignals innerhalb des Mediums mit erhöhter Genauigkeit und vergleichsweise geringem technischen Aufwand ermöglicht.

Die erfindungsgemäße Vorrichtung, bei der die zuvor hergeleitete und aufgezeigte Aufgabe gelöst ist, ist zunächst und im Wesentlichen dadurch gekennzeichnet, dass wenigstens eine Polarisationsvorrichtung zwischen der Sendevorrichtung und der Empfangsvorrichtung angeordnet ist, das Nutzsignal zumindest mittelbar von der Sendevorrichtung durch das mehrphasige Medium auf die Polarisationsvorrichtung emittiert wird, die Polarisationsvorrichtung das Nutzsignal in seiner Polarisation beeinflusst, und das polarisationsbeeinflusste Nutzsignal von der Polarisationsvorrichtung zumindest mittelbar zur Empfangsvorrichtung emittiert wird, wobei die Empfangsvorrichtung das polarisationsbeeinflusste Nutzsignal empfängt.

Die erfindungsgemäße Vorrichtung ist in vielerlei Hinsicht vorteilhaft. Zum einen wird durch die Polarisationsvorrichtung bewirkt, dass das interessierende Nutzsignal durch die bewirkte Polarisation gekennzeichnet und damit gegenüber anderen aber nicht interessierenden Signalen - Störsignalen - unterscheidbar gemacht wird, was grundsätzlich eine bessere Auswertung des durch die Empfangsvorrichtung empfangenen polarisationsbeeinflussten Nutzsignal ermöglicht. Dies ist ein großer Vorteil gegenüber aus dem Stand der Technik bekannten Transmissionsmessungen ohne den Zwischenschritt der Polarisationsbeeinflussung des in das mehrphasige Medium emittierten Nutzsignals. Vorausgesetzt wird allerdings, dass das durch die Empfangsvorrichtung empfangene Nutzsignal auch hinsichtlich seiner Polarisation ausgewertet werden kann, also insgesamt vektoriell ausgewertet werden kann, wobei es nicht entscheidend ist, nach welcher Methode eine solche Auswertung erfolgt; es können verschiedene aus dem Stand der Technik bekannte Methoden im Zeit- oder auch im Frequenzbereich angewendet werden, um eine Änderung der Polarisation - also beispielsweise einen Phasensprung - zu erkennen und damit das Nutzsignal von anderen Signalen zu unterscheiden.

Ein weiterer Vorteil der erfindungsgemäßen Vorrichtung besteht darin, dass das emittierte Nutzsignal das das mehrphasige Medium enthaltende Volumen nicht nur einmal durchläuft, sondern das Volumen aufgrund der Zwischenschaltung der Polarisationsvorrichtung auch mehrfach durchlaufen kann. Deshalb ist gemäß einer vorteilhaften Weiterbildung der erfindungsgemäßen Vorrichtung vorgesehen, dass die wenigstens eine Polarisationsvorrichtung so angeordnet ist, dass das Nutzsignal das das mehrphasige Medium enthaltende Volumen möglichst vollständig durchläuft, insbesondere vollständig durchläuft sowohl auf dem Weg von der Sendevorrichtung zur Polarisationsvorrichtung als auch auf dem Weg von der Polarisationsvorrichtung zur Empfangsvorrichtung. Dabei ist es durchaus möglich, dass die beiden Wege nicht direkt, sondern auch auf Umwegen durchlaufen werden, beispielsweise durch Zwischenschaltung weiterer Polarisationsvorrichtungen. Das das mehrphasige Medium enthaltende Volumen kann beispielsweise durch eine Röhre begrenzt sein, durch die das mehrphasige Medium strömt. In diesen Fällen wird das Nutzsignal bevorzugt senkrecht zur Strömungsrichtung emittiert, wobei möglichst große Durchmesser von dem Nutzsignal durchlaufen werden.

Bei einem gerätetechnisch besonders einfachen Ausführungsbeispiel ist vorgesehen, dass die Polarisationsvorrichtung ein passiver Polarisationsreflektor ist, die Sendevorrichtung das Nutzsignal in einer ersten Richtung polarisiert emittiert, der passive Polarisationsreflektor die Polarisation des Nutzsignals in einer von der ersten Richtung verschiedene zweite Richtung dreht und das Nutzsignal weiter emittiert, und die Empfangsvorrichtung das in der zweiten Richtung polarisierte Nutzsignal selektiv in der zweiten Richtung empfängt. Die Sendevorrichtung und die Empfangsvorrichtung sind dabei so ausgerichtet, dass entsprechend lange Laufwege des Nutzsignals resultieren.

Indem die Empfangsvorrichtung so eingerichtet ist, dass sie besonders empfindlich für ein in die zweite Richtung polarisiertes Nutzsignal ist - dieses also selektiv wahrnimmt - wird gewährleistet, dass andere nicht interessierende Signale - Störsignale -, die beispielsweise durch Störreflexionen des Nutzsignals an Gehäusewandungen resultieren, von der Empfangsvorrichtung aufgrund der im Regelfall andersartigen Polarisation nur gedämpft wahrgenommen werden.

Konstruktiv lässt sich das vorgenannte Ausführungsbeispiel besonders einfach dadurch realisieren, dass die Sendevorrichtung und die Empfangsvorrichtung mit einer gemeinsamen Antenne ausgebildet sind, die demnach sowohl zur Aussendung wie auch zum Empfang des Nutzsignals dient. Eine solche Antenne kann beispielsweise als Hohlleiter oder als Hornstrahler ausgestaltet sein, wobei das emittierte Nutzsignal dann üblicherweise linear polarisiert ist. In diesem Fall kann die Aussendung des Nutzsignals in einer ersten Polarisationsrichtung und der Empfang in einer sich davon unterscheidenden zweiten Polarisationsrichtung dadurch realisiert werden, dass in einem Hohlleiter der Antenne verschiedene Pfade für die Sendevorrichtung einerseits und die Empfangsvorrichtung andererseits ausgebildet sind, wobei die Orientierung dieser Pfade den gewünschten Polarisationsrichtungen des emittierten und des empfangenen Nutzsignals entsprechen. Der passive Polarisationsreflektor kann beispielsweise ein Kreuzpolarisationsreflektor sein, der eine Drehung der Polarisationsrichtung um 90° bewirkt, so dass auch die Pfade für die Sendevorrichtung einerseits und die Empfangsvorrichtung andererseits um 90° zueinander versetzt sind.

Eine weitere bevorzugte Ausgestaltung der Erfindung unterscheidet sich von dem zuvor erläuterten Ausführungsbeispiel dadurch, dass die Polarisationsvorrichtung kein passiver Polarisationsreflektor ist, sondern als eine aktive Polarisationsvonrichtung ausgestaltet ist, die Sendevorrichtung das Nutzsignal in einer ersten Richtung polarisiert emittiert, die aktive Polarisationsvorrichtung das Nutzsignal in eine zweite Richtung dreht und emittiert, und die Empfangsvorrichtung das in die zweite Richtung polarisierte Nutzsignal empfängt und die Auswertevorrichtung das empfangene Gesamtsignal vektoriell auswertet, so dass das in die zweite Richtung polarisierte Nutzsignal von anderen Anteilen des empfangenen Gesamtsignals unterscheidbar ist, insbesondere von Störsignalen. Auch bei diesem Ausführungsbeispiel wird das interessierende Nutzsignal durch die Polarisation gekennzeichnet und damit von nicht diese Polarisation aufweisender Störstrahlung unterscheidbar gemacht. Darüber hinaus wird auch hier das mit dem mehrphasigen Medium erfüllte Volumen zweimalig durchlaufen, so dass eine gegenüber der Ein-Weg-Transmissionmessung verdoppelte Wegstrecke zur Verfügung steht und damit der relative Messfehler geringer ist. Mit der vektoriellen Auswertung des empfangenen Gesamtsignals ist - wie schon zuvor erläutert - gemeint, dass das empfangene Signal nach Betrag und Phase ausgewertet werden kann, so dass verschieden polarisierte Anteile voneinander unterschieden werden können.

Passive und aktive Polarisationsvorrichtungen können auch gewinnbringend kombiniert werden, so dass sich eine weiter bevorzugte Ausgestaltung der erfindungsgemäßen Vorrichtung dadurch auszeichnet, dass eine erste, passive Polarisationsvorrichtung und eine zweite, aktive Polarisationsvorrichtung vorgesehen sind, die Sendevorrichtung und die Empfangsvorrichtung eine erste, also gemeinsame Polarisationsausrichtung aufweisen und die zweite, aktive Polarisationsvorrichtung eine von der ersten Polarisationsausrichtung abweichende zweite Polarisationsausrichtung aufweist. Eine solche Vorrichtung ist dann so ausgestaltet, dass die Sendevorrichtung das Nutzsignal zunächst in der ersten Polarisationsrichtung polarisiert und durch das Medium emittiert und die erste, also passive Polarisationsvorrichtung das Nutzsignal dann in die zweite Polarisationsausrichtung dreht und durch das Medium emittiert. Dieses in der zweiten Polarisationsausrichtung schwingende Nutzsignal kann selbst dann, wenn es in Richtung auf die Empfangsvorrichtung reflektiert werden sollte, die Empfangsvorrichtung nur in geringer Weise beeinflussen, da die Empfangsvorrichtung definitionsgemäß in dieser Polarisationsrichtung nicht sensitiv ist.

Die Vorrichtung ist dann ferner so ausgestaltet, dass die zweite, aktive Polarisationsvorrichtung das Nutzsignal dreht und durch das Medium wiederum emittiert und die erste, passive Polarisationsvorrichtung dieses Nutzsignal dann wiederum in die erste Polarisationsrichtung dreht und durch das Medium weiter emittiert, woraufhin die Empfangsvorrichtung das Nutzsignal empfängt und die Auswertevorrichtung das empfangene Gesamtsignal vektoriell auswertet, so dass das in die zweite Richtung polarisierte Nutzsignal von anderen Anteilen des empfangenen Gesamtsignals unterscheidbar ist. In einem konkreten Beispiel ist es also möglich, dass die Sendevorrichtung ein vertikal polarisiertes Nutzsignal emittiert, das von der ersten passiven Polarisationsvorrichtung um 90° gedreht wird, demnach also praktisch nicht von der Empfangsvorrichtung empfangbar ist, die zweite, aktive Polarisationsvorrichtung des Nutzsignals dann beispielsweise um 180° dreht - Reflexionsfaktor -1 -, das Signal dann zur ersten, passiven Polarisationsvorrichtung emittiert wird, dort eine weitere Drehung um 90° erfährt und mithin wieder von der Empfangsvorrichtung empfangbar ist. Bei einer solchen Anordnung durchläuft das Nutzsignal das das Medium führende Volumen sogar viermal, woraus sich eine entsprechende Verminderung des Messfehlers ergibt. Der Vorteil der Kennzeichnung des interessierenden Nutzsignals gegenüber den nicht-interessierenden und zu unterdrückenden Störsignalen wird darüber hinaus erreicht

Die Erfindung betrifft ferner auch ein Verfahren zum Betreiben einer der vorgenannten Vorrichtungen zur Bestimmung des Volumenanteils wenigstens einer Komponente eines mehrphasigen Mediums auf Grundlage der Laufzeit eines in das mehrphasige Medium emittierten elektromagnetischen Nutzsignals, wobei das Verfahren nur solche Vorrichtungen betrifft, die wenigstens eine aktive Polarisationsvorrichtung aufweisen, also eine solche Polarisationsvorrichtung, der vorgebbar ist, ob sie eine bestimmte Polarisation vornimmt oder eben keine Polarisation vornimmt, oder eine solche Polarisationsvorrichtung, der beliebige Polarisationsbeeinflussungen vorgebbar sind, die also nicht nur ein- bzw. abschaltbar ist.

Bei dem erfindungsgemäßen Verfahren wird bei einer ersten Messung bei der aktiven Polarisationsvorrichtung eine erste Polarisationsbeeinflussung eingestellt und ein erstes empfangenes Signal von der Empfangsvorrichtung erfasst. Nachfolgend wird bei einer zweiten Messung bei der aktiven Polarisationsvorrichtung eine von der ersten Polarisationsbeeinflussung verschiedene zweite Polarisationsbeeinflussung eingestellt und ein zweites empfangenes Signal erfasst. Es liegen demnach empfangene Signale mit verschiedenen polarisierten Nutzsignalen vor. Nachfolgend werden das erste empfangene Signal und das zweite empfangene Signal so überlagert, dass sich die in dem ersten empfangenen Signal und die in dem zweiten empfangenen Signal enthaltenen Störsignale aufheben, insbesondere sich sogar die in dem ersten empfangenen Signal und die in dem zweiten empfangenen Signal enthaltenen Nutzsignale auch addieren. Besonders einfach vorstellbar ist dies beispielsweise bei einer ersten Messung, bei der die aktive Polarisationsvorrichtung deaktiviert wird, also die erste Polarisationsbeeinflussung in einer Nicht-Beeinflussung realisiert ist, wobei bei der zweiten Messung die aktive Polarisationsvorrichtung so eingestellt wird, dass ein negativer Reflexionsfaktor realisiert ist, also ein Phasensprung des Nutzsignals um 180° realisiert ist. Wenn der Anteil des empfangenen Gesamtsignals, der diesen Phasensprung aufweist, der demzufolge von dem interessierenden Nutzsignal stammt, mit einem negativen Vorzeichen behaftet wird und das erste empfangene Signal und das so beeinflusste zweite empfangene Signal nachfolgend voneinander abgezogen werden, heben sich praktisch die in den empfangenen Signalen enthaltenen Störsignalen auf - vorausgesetzt sie sind von gleicher Struktur, weil sie beispielsweise von festen Reflexionsflächen stammen - während sich die mit unterschiedlichen Vorzeichen behafteten Nutzsignale addieren, was die Erkennung des Nutzsignals insgesamt vereinfacht und damit die Bestimmung der Signallaufzeit.

Im Einzelnen gibt es nun verschiedene Möglichkeiten, die erfindungsgemäße Vorrichtung und das erfindungsgemäße Verfahren auszugestalten und weiterzubilden. Dazu wird verwiesen auf die den Patentansprüchen 1 und 10 nachgeordneten Patentansprüche und auf die Beschreibung bevorzugter Ausführungsbeispiele in Verbindung mit der Zeichnung. In der Zeichnung zeigen

Fig. 1    eine Vorrichtung zur Bestimmung des Volumenanteils wenigstens einer Komponente eines mehrphasigen Mediums gemäß dem Stand der Technik,

Fig. 2    eine erfindungsgemäße Vorrichtung in schematischer Darstellung mit zwei Messwegen und einer passiven Polarisationsvorrichtung,

Fig. 3    ein weiteres Ausführungsbeispiel einer erfindungsgemäßen Vorrichtung mit einer aktiven Polarisationsvorrichtung und zwei Messwegen,

Fig. 4    ein weiteres Ausführungsbeispiel einer erfindungsgemäßen Vorrichtung mit einer passiven Polarisationsvorrichtung und einer aktiven Polarisationsvorrichtung und insgesamt vier Messwegen,

Fig. 5    ein weiteres Ausführungsbeispiel einer erfindungsgemäßen Vorrichtung, wobei Sendevorrichtung, Empfangsvorrichtung und Polarisationsvorrichtung in einem Gerät zusammenfallen und

Fig. 6    ein weiteres Ausführungsbeispiel einer erfindungsgemäßen Vorrichtung mit vier Messwegen.

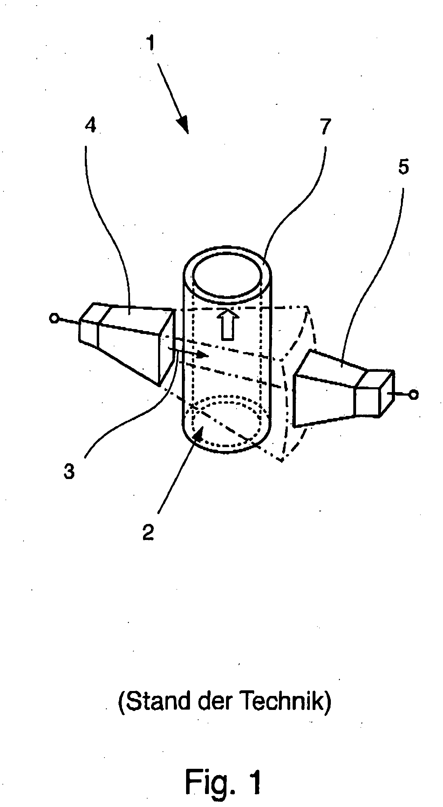

In Fig. 1 ist dargestellt eine Vorrichtung 1 zur Bestimmung des Volumenanteils wenigstens einer Komponente eines mehrphasigen Mediums 2 auf Grundlage der Laufzeit eines in das mehrphasige Medium 2 emittierten elektromagnetischen Nutzsignals 3, mit einer Sendevorrichtung 4 zur Emittierung des Nutzsignals 3 in das mehrphasige Medium 2, mit einer Empfangsvorrichtung 5 zum Empfangen des Nutzsignals 3 und einer hier nicht dargestellten, in anderen Figuren mit dem Bezugszeichen 6 versehenen Auswertevorrichtung zur Bestimmung der Laufzeit des Nutzsignals 3 zwischen der Sendevorrichtung 4 und der Empfangsvorrichtung 5.

Im vorliegenden Fall wird das mehrphasige Medium 2 in einem Rohr 7 geführt, wobei sich das Medium 2 in die mit dem Pfeil angedeutete Richtung bewegt. Im Stand der Technik wird das von der Sendevorrichtung 4 emittierte Nutzsignal 3 und das von der Empfangsvorrichtung 5 empfangene Nutzsignal 3 über Hochfrequenzleitungen einem Netzwerkanalysator zugeführt, wodurch Rückschlüsse auf das Übertragungsverhalten der Gesamtstrecke und damit auch auf die Laufzeit des Nutzsignals 3 zwischen der Sendevorrichtung 4 und der Empfangsvorrichtung 5 gezogen werden können. Problematisch ist, dass das von der Empfangsvorrichtung 5 empfangene Gresamtsignal nicht nur das interessierende Nutzsignal 3, sondern vielmehr auch Störsignale enthält, die Unterscheidung von Nutzsignal und Störsignal einen hohen Aufwand erfordert, teilweise nicht möglich ist und insgesamt der gerätemäßige Aufwand erheblich ist und in der industriellen Praxis im Prozess nicht erbracht werden kann.

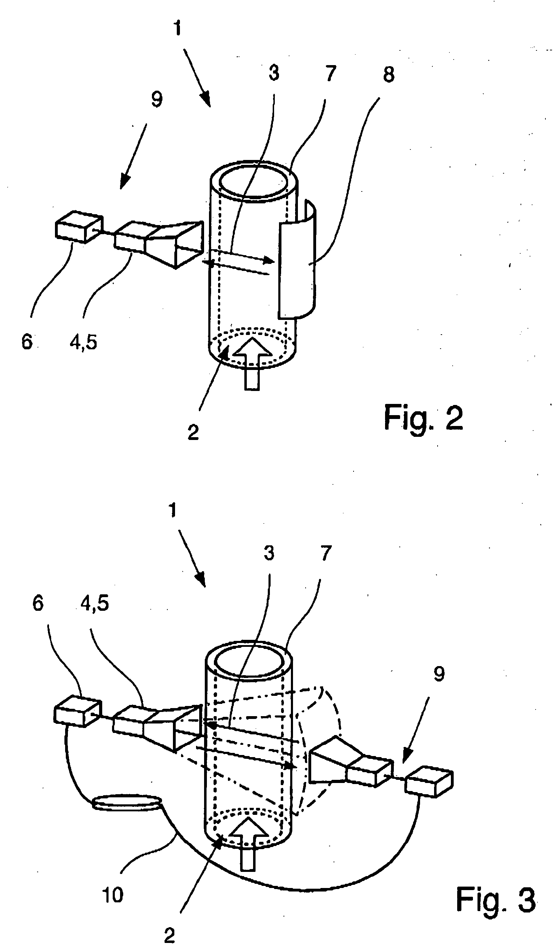

Dem in den Fig. 2 bis 6 dargestellten Lösungen ist gemein, dass zumindest eine Polarisationsvorrichtung 8, 9 zwischen der Sendevorrichtung 4 und der Empfangsvorrichtung 5 angeordnet ist, das Nutzsignal 3 von der Sendevorrichtung 4 durch das mehrphasige Medium 2 auf die Polarisationsvorrichtung 8, 9 emittiert wird, die Polarisationsvorrichtung 8, 9 das Nutzsignal 3 in seiner Polarisation beeinflusst und das polarisationsbeeinflusste Nutzsignal 3 durch das Medium von der Polarisationsvorrichtung 8, 9 zur Empfangsvorrichtung 5 emittiert, wobei die Empfangsvorrichtung 5 das polarisationsbeeinflusste und damit quasi individualisierte Nutzsignal 3 empfängt. Vor dem Empfangen des Nutzsignals 3 durch die Empfangsvorrichtung 5 wird das Nutzsignal 3 durch die Polarisation unterscheidbar gemacht von anderen in dem empfangenen Gesamtsignal enthaltenen Signalen, also auch von Störsignalen. Wie in sämtlichen Figuren zu erkennen ist, sind die Polarisationsvorrichtung 8 bzw. die Polarisationsvorrichtung 9 bzw. die Polarisationsvorrichtungen 8 und 9 so angeordnet, dass das Nutzsignal 3 das das mehrphasige Medium 2 enthaltende Rohr 7 möglichst vollständig durchläuft, sowohl auf dem Weg von der Sendevorrichtung 4 zur Polarisationsvorrichtung 8, 9 als auch auf dem Weg von der Polarisationsvorrichtung 8, 9 zur Empfangsvorrichtung 5. Das Nutzsignal 3 durchläuft bei der erfindungsgemäßen Vorrichtung demnach das das mehrphasige Medium 2 enthaltende Volumen zumindest zweimal oder sogar öfter, was den Messfehler entsprechend verringert. Das Nutzsignal 3 wird insbesondere so emittiert, das es senkrecht zur Strömungsrichtung verläuft.

Bei dem Ausführungsbeispiel gemäß Fig. 2 ist die Polarisationsvorrichtung 8 ein passiver Polarisationsreflektor. Die Sendevorrichtung 4 emittiert das Nutzsignal 3 polarisiert in einer ersten Richtung, der passive Polarisationsreflektor 8 dreht die Polarisation des Nutzsignals 3 in eine von der ersten Richtung verschiedene zweite Richtung, vorliegend nämlich um 90°, und emittiert das Nutzsignal 3 weiter. Die Empfangsvorrichtung 5 ist in Baueinheit mit der Sendevorrichtung 4 ausgebildet und ist so ausgestaltet, dass sie das in die zweite Richtung polarisierte Nutzsignal selektiv in dieser zweiten Richtung empfängt. Die unterschiedlichen Polarisationsrichtungen der Sendevorrichtung 4 und der Empfangsvorrichtung 5 sind in dem dargestellten Ausführungsbeispiel so realisiert, dass in einem Hohlleiter der dargestellten Antenne verschiedene Pfade für die Sendevorrichtung 4 einerseits und die Empfangsvorrichtung 5 andererseits ausgebildet sind, entsprechend den 90° zueinander stehenden Polarisationsrichtungen von emittiertem und empfangenem Nutzsignal 3. Bei dem passiven Polarisationsreflektor 8 handelt es sich vorliegend um einen Kreuzpolarisationsreflektor.

Bei dem in Fig. 3 dargestellten Ausführungsbeispiel ist die Polarisationsvorrichtung 9 realisiert durch eine aktive Polarisationsvorrichtung 9, der also eine bestimmte Polarisation bzw. eine bestimmte Polarisationsänderung vorgebbar ist, bzw. die von ihrer Polarisationsbeeinflussung her ein- und abschaltbar ist. Zu diesem Zweck ist die Polarisationsvorrichtung 9 mit einem Niederfrequenz-Kabel 10 mit der Auswertevorrichtung 6 verbunden, die diese Steuerungsaufgabe übernimmt. Die aktive Polarisationsvorrichtung 9 ist hier realisiert durch einen Modulator mit umschaltbarem Reflexionskoeffizienten.

Die Sendevorrichtung 4 emittiert das Nutzsignal 3 polarisiert in einer ersten Richtung. Die aktive Polarisationsvorrichtung 9 dreht das Nutzsignal 3 dann in eine zweite Richtung und emittiert das Nutzsignal 3 weiter, nämlich zurück in Richtung auf die Empfangsvorrichtung 5, die das in zweite Richtung polarisierte Nutzsignal 3 empfängt, wobei die Auswertevorrichtung 6 das empfangene Gesamtsignal nachfolgend vektoriell auswertet, so dass das in die zweite Richtung polarisierte Nutzsignal 3 von anderen Anteilen des empfangenen Gesamtsignals unterscheidbar ist, insbesondere unterscheidbar ist von den empfangenen Störsignalen. Auch hier wird das mit dem mehrphasigen Medium 2 erfüllte Volumen in Form des Rohres 7 zweimalig von dem Nutzsignal 3 durchlaufen. Die Umschaltung der aktiven Polarisationsvorrichtung 9 mit dem Niederfrequenz-Kabel 10 ist einfach und preiswert, denn es müssen keine zeitkritischen Vorgänge im Bereich der Laufzeit des Nutzsignals 3 berücksichtigt werden.

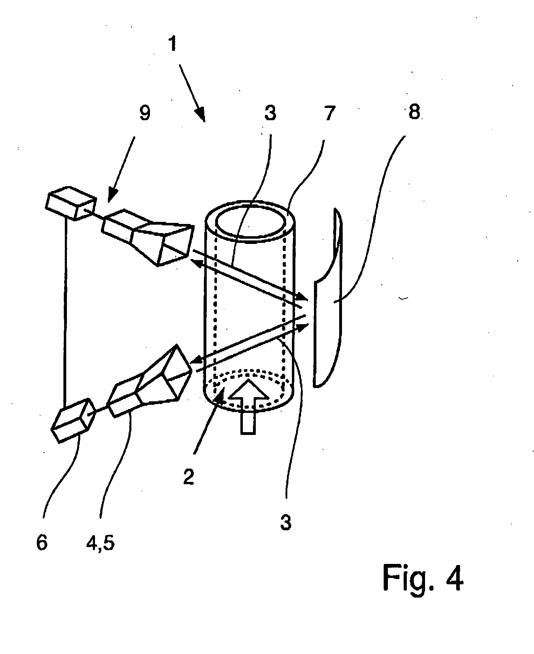

Bei dem Ausführungsbeispiel gemäß Fig. 4 ist sowohl eine erste passive Polarisationsvorrichtung 8 als auch eine zweite, aktive Polarisationsvorrichtung 9 vorgesehen. Die Sendevorrichtung 4 und die Empfangsvorrichtung 5 weisen eine erste Polarisationsausrichtung auf, die zweite, aktive Polarisationsvorrichtung 9 weist eine von der ersten Polarisationsausrichtung abweichende zweite Polarisationsausrichtung auf; vorliegend sind die Polarisationsausrichtungen um 90° gegeneinander gedreht. Die Sendevorrichtung 4 sendet das Nutzsignal 3 in der ersten Polarisationsausrichtung polarisiert in das Medium 2 aus. Die erste, passive Polarisationsvorrichtung 8 dreht das Nutzsignal 3 um 90° in die zweite Polarisationsausrichtung und emittiert das so polarisationsbeeinflusste Nutzsignal 3 weiter in das Medium 2. Dieses emittierte Nutzsignal 3 kann die Empfangsvorrichtung 5 nicht stören, da die Empfangsvorrichtung 5 in dieser Polarisationsrichtung nicht empfindlich ist. Die zweite, aktive Polarisationsausrichtung 9 ist als Modulator ausgestaltet und bewirkt eine Phasendrehung des empfangenen Nutzsignals 3 um 180° und eine darauffolgende weitere Emittierung des Nutzsignals 3 durch das Medium 2. Das so polarisationsbeeinflusste Nutzsignal 3 ist für die Empfangsvorrichtung 5 immer noch nicht empfangbar und stört die Empfangsvorrichtung 5 damit auch nicht. Das Nutzsignal 3 wird letztlich durch die erste, passive Polarisationsvorrichtung ein weiteres Mal um 90° gedreht in die erste Polarisationsausrichtung und wird von dort aus ein weiteres Mal in das Medium 2 emittiert und ist nachfolgend von der Empfangsvorrichtung 5 empfangbar, wobei die Auswertevorrichtung 6 das empfangene Gesamtsignal wiederum vektoriell auswertet mit dem Ziel, dass das in die zweite Richtung polarisierte Nutzsignal 3 von anderen Anteilen des empfangenen Gesamtsignals unterscheidbar ist und unterschieden wird. Bei dieser Lösung liegt das Nutzsignal insgesamt vier Wege durch das Medium 2 zurück mit den beschriebenen positiven Effekten auf die Messgenauigkeit.

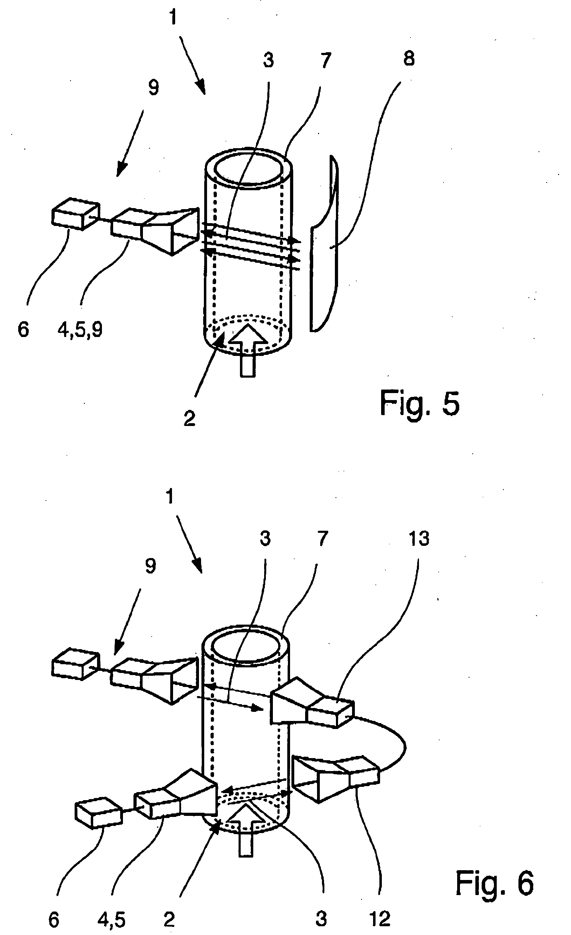

Das Ausführungsbeispiel gemäß Fig. 5 ist funktional ähnlich zu dem Ausführungsbeispiel gemäß Fig. 4, wobei die Sendevorrichtung 4, die Empfangsvorrichtung 5 und die zweite, aktive Polarisationsvorrichtung 9 mit einer gemeinsamen Antenne ausgebildet sind. Die Sendevorrichtung 4 und die Empfangsvorrichtung 5 weisen eine erste gemeinsame Polarisationsausrichtung auf und die zweite aktive Polarisationsvorrichtung 9 weist eine zweite, von der ersten Polarisationsausrichtung verschiedene Polarisationsausrichtung auf. Das Funktionsprinzip ist ähnlich wie bei dem Ausführungsbeispiel gemäß Fig. 4, jedoch ist die Bauweise aufgrund der gerätetechnischen Zusammenfassung wesentlich kompakter.

Das Ausführungsbeispiel gemäß Fig. 6 ist funktional recht ähnlich dem Ausführungsbeispiel gemäß Fig. 3, wobei der Messpfad verlängert wird durch die Phasenlage und die Polarisation nicht beeinflussende Antennen 12 und 13. Die Messpfade zwischen der Sendevorrichtung 4 bzw. der Empfangsvorrichtung 5 und der Antenne 12 einerseits sowie zwischen der Antenne 13 und der zweiten aktiven Polarisationsvorrichtung 9 andererseits sind um 90° gegeneinander gedreht - gedachte Drehachse das Rohr 7 - und in Verlaufsrichtung des Rohres voneinander beabstandet. Vorteil ist hier, dass durch die geometrische Trennung der Antennenpaare 4, 5 und 12 einerseits und 13 und 9 andererseits, eine Mittelung über zwei verschiedene Rohrquerschnitte erfolgt, was ebenfalls die Messgenauigkeit erhöht.

In den dargestellten Ausführungsbeispielen gemäß den Fig. 3 bis 6, die also eine aktive Polarisationsvorrichtung 9 aufweisen, ist die Auswertevorrichtung 6 so ausgestaltet, dass mit ihr das nachfolgend beschriebene Verfahren zum Betreiben der dargestellten Vorrichtungen durchgeführt werden kann. Demnach wird bei einer ersten Messung bei der aktiven Polarisationsvorrichtung 9 eine erste Polarisationsbeeinflussung eingestellt, die vorliegend darin besteht, dass das Nutzsignal 3 einfach reflektiert wird. Die Empfangsvorrichtung 5 erfasst das erste empfangene Signal. Bei der zweiten Messung wird der aktiven Polarisationsvorrichtung 9 eine von der ersten Polarisationsbeeinflussung verschiedene zweite Polarisationsbeeinflussung aufgegeben, vorliegend wird ein negativer Reflexionsfaktor eingestellt und von der Empfangsvorrichtung 5 wird ein zweites empfangenes Signal erfasst. Die Auswertevorrichtung 6 verrechnet das erste empfangene Signal und das zweite empfangene Signal derart, dass sich die in dem ersten empfangenen Signal und die in dem zweiten empfangenen Signal enthaltenen Störsignale - zumindest größtenteils, insofern sie ähnlich sind - aufheben, wodurch das Nutzsignal 3 in dem Gesamtsignal wesentlich sicherer detektiert werden kann und dadurch auch die Laufzeitmessung des Nutzsignals an Qualität gewinnt.

Genauer wird also bei der ersten Messung bei der zweiten Polarisationsvorrichtung 9 ein positiver Reflexionskoeffizient eingestellt und wird bei der zweiten Messung bei der zweiten Polarisationsvorrichtung 9 ein negativer Reflexionskoeffizient eingestellt, wobei der Phasenwechsel des Nutzsignals bei der zweiten Messung erkannt und vorzeichenmässig im zweiten erfassten Signal berücksichtigt wird. Dadurch ist es möglich, dass bei Überlagerung der beiden empfangenen Signale sich die Störsignale aufheben und sich die Nutzsignale addieren.

**Patentansprüche**

1. Vorrichtung (1) zur Bestimmung des Volumenanteils wenigstens einer Komponente eines mehrphasigen Mediums (2) auf Grundlage der Laufzeit eines in das mehrphasige Medium (2) emittierten elektromagnetischen Nutzsignals (3), mit wenigstens einer Sendevorrichtung (4) zur Emittierung des Nutzsignals (3) in das mehrphasige Medium (2), mit wenigstens einer Empfangsvorrichtung (5) zum Empfang des Nutzsignals (3) und mit einer Auswertevorrichtung (6) zur Bestimmung der Laufzeit des Nutzsignals (3) zwischen der Sendevorrichtung (4) und der Empfangsvorrichtung (5),
**dadurch gekennzeichnet,**
**dass** wenigstens eine Polarisationsvorrichtung (8, 9) zwischen der Sendevorrichtung (4) und der Empfangsvorrichtung (5) angeordnet ist, das Nutzsignal (3) zumindest mittelbar von der Sendevorrichtung (4) durch das mehrphasige Medium (2) auf die Polarisations Vorrichtung (8, 9) emittiert wird, die Polarisationsvorrichtung (8, 9) dazu eingerichtet ist, das Nutzsignal (3) in seiner Polarisation zu beeinflussen und das polarisationsbeeinflusste Nutzsignal (3) zumindest mittelbar von der Polarisationsvorrichtung (8, 9) zur Empfangsvorrichtung (5) zu emittieren, wobei die Empfangsvorrichtung (5) dazu eingerichtet ist, das polarisationsbeeinflusste Nutzsignal (3) zu empfangen und die Auswertevorrichtung (6) dazu eingerichtet ist, ein empfangenes Gesamtsignal vektoriell hinsichtlich der Polarisation des empfangenen Nutzsignals (3) auszu werten, um das empfangene Nutzsignal (3) von anderen Signalen des empfangenen Gesamtsignals zu unterscheiden.

2. Vorrichtung (1) nach Anspruch 1, **dadurch gekennzeichnet, dass** die wenigstens eine Polarisationsvorrichtung (8, 9) so angeordnet ist, dass das Nutzsignal (3) das das mehrphasige Medium (2) enthaltende Volumen möglichst vollständig durchläuft, insbesondere vollständig durchläuft sowohl auf dem Weg von der Sendevorrichtung (4) zur Polarisationsvorrichtung (8, 9) als auch auf dem Weg von der Polarisationsvorrichtung (8, 9) zur Empfangsvorrichtung (5).

3. Vorrichtung (1) nach Anspruch 1 oder 2, **dadurch gekennzeichnet, dass** die Polarisationsvorrichtung (8) ein passiver Polarisationsreflektor ist, die Sendevorrichtung (5) dazu eingerichtet ist, das Nutzsignal (3) in einer ersten Richtung zu polarisier in emittiert, der passive Polarisationsreflektor (8) dazu eingerichtet ist, die Polarisation des Nutzsignals (3) in eine von der ersten Richtung verschiedene zweite Richtung zu drehen und das Nutzsignal (3) weiter zu emittieren, und die Empfangsvorrichtung (5) dazu eingerichtet ist, das in die zweite Richtung polarisierte Nutzsignal (3) selektiv in der zweiten Richtung zu empfangen.

4. Vorrichtung (1) nach Anspruch 3, **dadurch gekennzeichnet, dass** die Sendevorrichtung (4) und die Empfangsvorrichtung (5) mit einer gemeinsamen Antenne ausgebildet sind, insbesondere in einem Hohlleiter der Antenne verschiedene Pfade für die Sendevorrichtung (4) einerseits und die Empfangsvorrichtung (5) andererseits ausgebildet sind, entsprechend den Polarisationsrichtungen von emittiertem und empfangenem Nutzsignal (3).

5. Vorrichtung (1) nach Anspruch 1 oder 2, **dadurch gekennzeichnet, dass** die Polarisationsvorrichtung (9) eine aktive Polarisationsvorrichtung ist, die Sendevorrichtung (4) dazu eingerichtet ist, das Nutzsignal (3) in einer ersten Richtung polarisiert zu emittieren, die aktive Polarisationsvorrichtung (9) dazu eingerichtet ist, das Nutzsignal (3) in eine zweite Richtung zu drehen und zu emittieren, und die Empfangsvorrichtung (5) dazu eingerichtet ist, das in die zweite Richtung polarisierte Nutzsignal (3) zu empfangen und die Auswertevorrichtung (6) dazu eingerichtet ist, das empfangene Gesamtsignal vektoriell aus zu werten, so dass das in die zweite Richtung polarisierte Nutzsignal (3) von anderen Anteilen des empfangenen Gesamtsignals unterscheidbar ist.

6. Vorrichtung nach Anspruch 1 oder 2, **dadurch gekennzeichnet, dass** eine erste, passive Polarisationsvorrichtung (8) und eine zweite, aktive Polarisationsvorrichtung (9) vorgesehen sind, die Sendevorrichtung (4) und die Empfangsvorrichtung (5) eine erste Polarisationsausrichtung aufweisen, die zweite, aktive Polarisationsvorrichtung (9) eine von der ersten Polarisationsausrichtung abweichende zweite Polarisationsausrichtung aufweist, die Sendevorrichtung (4) dazu eingerichtet ist, das Nutzsignal (3) in der ersten Polarisationsausrichtung zu polarisieren und durch das Medium (2) zu emittieren, die erste, passive Polarisationsvorrichtung (8) dazu eingerichtet ist, das Nutzsignal (3) in die zweite Polarisationsausrichtung zu drehen und durch das Medium (2) zu emittieren, die zweite, aktive Polarisationsvorrichtung (9) dazu eingerichtet ist, das Nutzsignal (3) zu drehen und durch das Medium (2) zu emittieren, die erste, passive Polarisations Vorrichtung (8) dazu eingerichtet ist, das Nutzsignal (3) in die erste Polarisationsausrichtung zu drehen und durch das Medium (2) zu emittieren, die Empfangsvorrichtung (4) dazu eingerichtet ist, das Nutzsignal (3) zu empfangen und die Auswertevorrichtung (6) dazu eingerichtet ist, das empfangene Gesamtsignal vektoriell auszuwerten, so dass das in die zweite Richtung polarisierte Nutzsignal (3) von anderen Anteilen des empfangenen Gesamtsignals unterscheidbar ist.

7. Vorrichtung (1) nach Anspruch 6, **dadurch gekennzeichnet, dass** die Sendevorrichtung (4), die Empfangsvorrichtung (5) und die zweite, aktive Polarisationsvorrichtung (9) mit einer gemeinsamen Antenne ausgebildet sind, die Sendevorrichtung (4) und die Empfangsvorrichtung (5) eine erste Polarisationsausrichtung aufweisen und die zweite, aktive Polarisationsvorrichtung (9) eine zweite, von der ersten Polarisationsausrichtung verschiedene Polarisationsausrichtung aufweist.

8. Vorrichtung (1) nach Anspruch 7, **dadurch gekennzeichnet, dass** insbesondere in einem Hohlleiter der gemeinsamen Antenne zwei verschiedene Pfade für die Sendevorrichtung (4) und die Empfangsvorrichtung (5) einerseits und für die zweite, aktive Polarisationsvorrichtung (9) andererseits ausgebildet sind, entsprechend den Polarisationsausrichtungen des von der Sendevorrichtung (4) emittierten und von der Empfangsvorrichtung (5) empfangenen Nutzsignals (3) einerseits und des von der zweiten, aktiven Polarisationsvorrichtung (9) emittierten und empfangenen Nutzsignals (3) andererseits.

9. Vorrichtung nach einem der Ansprüche 5 bis 8, **dadurch gekennzeichnet, dass** die aktive Polarisationsvorrichtung (9) als Modulator realisiert ist, insbesondere als Modulator zur Realisierung einstellbarer Reflexionskoeffizienten, bevorzugt auf Basis von Hochfrequenzschaltern.

10. Verfahren zum Betreiben einer Vorrichtung (1) zur Bestimmung des Volumenanteils wenigstens einer Komponente eines mehrphasigen Mediums (2) auf Grundlage der Laufzeit eines in das mehrphasige Medium (2) emittierten elektromagnetischen Nutzsignals (3) nach einem der Ansprüche 5 bis 9, mit wenigstens einer aktiven Polarisationsvorrichtung (9), wobei bei einer ersten Messung bei der aktiven Polarisationsvorrichtung (9) eine erste Polarisationsbeeinflussung eingestellt wird und von der Empfangsvorrichtung (5) ein erstes empfangenes Signal erfasst wird, bei einer zweiten Messung bei der aktiven Polarisationsvorrichtung (9) eine von der ersten Polarisationsbeeinflussung verschiedene zweite Polarisationsbeeinflussung eingestellt wird und ein zweites empfangenes Signal von der Empfangsvorrichtung (5) erfasst wird, und das erste empfangene Signal und das zweite empfangene Signal so überlagert werden, dass sich die in dem ersten empfangenen Signal und die in dem zweiten empfangenen Signal enthaltenen Störsignale aufheben, insbesondere sich auch die in dem ersten empfangenen Signal und die in dem zweiten empfangenen Signal enthaltenen Nutzsignale addieren.

11. Verfahren nach Anspruch 10, **dadurch gekennzeichnet, dass** bei der ersten Messung bei der zweiten, aktiven Polarisationsvorrichtung (9) ein positiver Reflexionskoeffizient eingestellt wird und bei der zweiten Messung bei der zweiten, aktiven Polarisationsvorrichtung (9) ein negativer Reflexionskoeffizient eingestellt wird, insbesondere wobei der Phasenwechsel des Nutzsignals bei der zweiten Messung erkannt und vorzeichenmäßig berücksichtigt wird.

## Claims

1. Device for determining the volume portion of at least one component of a multi-phase medium (2) on the basis of the transit time of an electromagnetic desired signal (3) emitted in the multi-phase medium (2), having at least one transmitting device (4) for emitting the desired signal (3) into the multi-phase medium (2), having at least one receiving device (5) for receiving the desired signal (3) and with an evaluation unit (6) for determining the transit time of the desired signal (3) between the transmitting device (4) and the receiving device (5),
**characterized in**
**that** at least one polarization device (8, 9) is arranged between the transmitting device (4) and the receiving device (5), the desired signal (3) is emitted at least indirectly by the transmitting device (4) through the multi-phase medium (2) to the polarization device (8, 9), the polarization device (8, 9) is designed to influence the desired signal (3) in its polarization and to emit the desired signal (3) influenced in its polarization at least indirectly from the polarization device (8, 9) to the receiving device (5), wherein the receiving device (5) is designed to receive the desired signal (3) influenced in its polarization and the evaluation unit (6) is designed to evaluate a received entire signal vectorially in view of the polarization of the received desired signal (3) in order to differentiate the received desired signal (3) from other signals of the received overall signal.

2. Device (1) according to claim 1, **characterized in that** the at least one polarization device (8, 9) is arranged in such a manner that the desired signal (3) completely traverses the volume containing the multi-phase medium (2), in particular completely traverses it on the path from the transmitting device (4) to the polarization device (8, 9) as well as on the path from the polarization device (8, 9) to the receiving device (5).

3. Device (1) according to claim 1 or 2, **characterized in that** the polarization device (8) is a passive polarization reflector, the transmitting device (5) is designed to emit the desired signal (3) polarized in a first direction, the passive polarization reflector (8) is designed to turn the polarization of the desired signal (1) in a second direction different from the first direction and to further emit the desired signal (3), and the receiving device (5) is designed to receive the desired signal (3) polarized in the second direction selectively in the second direction.

4. Device (1) according to claim 3, **characterized in that** the transmitting device (4) and the receiving device (5) are designed with a common antenna, in particular different paths for the transmitting device (4) on the one hand and the receiving device (5) on the other hand are designed in a waveguide of the antenna, in accordance with the polarization direction of the emitted and received desired signal (3).

5. Device (1) according to claim 1 or 2, **characterized in that** the polarization device (9) is an active polarization device, the transmitting device (4) is designed to emit the desired signal (3) polarized in a first direction, the active polarization device (9) is designed to turn and emit the desired signal (3) in a second direction, and the receiving device (5) is designed to receive the desired signal (3) polarized in the second direction and the evaluation unit (6) is designed to evaluate the received entire signal vectorially, so that the desired signal (3) polarized in the second direction can be differentiated from other portions of the received entire signal.

6. Device (1) according to claim 1 or 2, **characterized in that** a first, passive polarization device (8) and a second, active polarization device (9) are provided, the transmitting device (4) and the receiving device (5) have a first polarization direction, the second, active polarization device (9) has a second polarization direction differing from the first polarization direction, the transmitting device (4) is designed to polarize the desired signal (3) in the first polarization direction and emit it through the medium (2), the first, passive polarization device (8) is designed to turn the desired signal (3) in the second polarization direction and emit it through the medium (2), the second, active polarization unit (9) is designed to turn the desired signal (3) and emit it through the medium (2), the first, passive polarization device (8) is designed to turn the desired signal (3) in the first polarization direction and emit it through the medium (2), the receiving device (4) is designed to receive the desired signal (3) and the evaluation unit (6) is designed to evaluate the received entire signal vectorially, so that the desired signal (3) polarized in the second direction can be differentiated from the other portions of the received entire signal.

7. Device (1) according to claim 6, **characterized in that** the transmitting device (4), the receiving device (5) and the second, active polarization device (9) are designed with a common antenna, the transmitting device (4) and the receiving device (5) have a first polarization direction and the second, active polarization device (9) has a second polarization direction differing from the first polarization direction.

8. Device (1) according to claim 7, **characterized in that** two different paths for the transmitting device (4) and the receiving device (5) on the one hand and for the second, active polarization device (9) on the other hand are designed in particular in a waveguide of the common antenna, in accordance with the polarization direction of the desired signal (3) emitted from the transmitting device (4) and received by the receiving device (5) on the one hand and emitted by the second, active polarization device (9) and the received desired signal (3) on the other hand.

9. Device according to any one of claims 5 to 8, **characterized in that** the active polarization device (9) is implemented as a modulator, in particular as a modulator for implementing adjustable reflection coefficients, preferably on the basis of high frequency switches.

10. Method for operating a device (1) for determining the volume portion of at least one component of a multiphase medium (2) on the basis of the transit time of an electromagnetic desired signal (3) emitted in the multi-phase medium (2) according to any one of claims 5 to 9, having at least one active polarization device (9), wherein, during a first measurement, a first polarization influence is set by the active polarization device (9) and a first received signal is detected by the receiving device (5), during a second measurement, a second polarization influence differing from the first polarization influence is set by the active polarization device (9) and a second received signal is detected by the receiving device (5) and the first received signal and the second received signal are superimposed in such a manner that interferences in the first received signal and in the second received signal are cancelled out, in particular the desired signals in the first received signal and the second received signal are added together.

11. Method according to claim 10, **characterized in that**, during the first measurement, a positive reflection coefficient is set by the second, active polarization device (9) and, during the second measurement, a negative reflection coefficient is set by the second, active polarization device (9), in particular wherein the phase shift of the desired signal is recognized and taken into consideration in respect to its sign during the second measurement.

**Revendications**

1. Dispositif (1) destiné à déterminer la part volumique d'au moins un composant d'un fluide (2) à plusieurs phases en se basant sur le temps de propagation d'un signal utile (3) électromagnétique émis dans le fluide (2) à plusieurs phases, comprenant au moins un dispositif d'émission (4) servant à émettre le signal utile (3) dans le fluide (2) à plusieurs phases, comprenant au moins un dispositif de réception (5) destiné à recevoir le signal utile (3) et comprenant un dispositif d'interprétation (6) destiné à déterminer le temps de propagation du signal utile (3) entre le dispositif d'émission (4) et le dispositif de réception (5),
**caractérisé en ce**
**qu'**au moins un dispositif de polarisation (8, 9) est disposé entre le dispositif d'émission (4) et le dispositif de réception (5), le signal utile (3) est émis au moins indirectement par le dispositif d'émission (4) à travers le fluide (2) à plusieurs phases sur le dispositif de polarisation (8, 9), le dispositif de polarisation (8, 9) est conçu pour influencer la polarisation du signal utile (3) et pour émettre le signal utile (3) à la polarisation influencée au moins indirectement du dispositif de polarisation (8, 9) vers le dispositif de réception (5), le dispositif de réception (5) étant conçu pour recevoir le signal utile (3) à la polarisation influencée et le dispositif d'interprétation (6) étant conçu pour effectuer une interprétation vectorielle d'un signal total reçu du point de vue de la polarisation du signal utile (3) reçu afin de différencier le signal utile (3) reçu d'autres signaux du signal total reçu.

2. Dispositif (1) selon la revendication 1, **caractérisé en ce que** l'au moins un dispositif de polarisation (8, 9) est disposé de telle sorte que le signal utile (3) traverse autant que possible totalement le volume qui contient le fluide (2) à plusieurs phases, notamment le traverse totalement sur le chemin du dispositif d'émission (4) vers le dispositif de polarisation (8, 9) ainsi que sur le chemin du dispositif de polarisation (8, 9) vers le dispositif de réception (5).

3. Dispositif (1) selon la revendication 1 ou 2, **caractérisé en ce que** le dispositif de polarisation (8) est un réflecteur de polarisation passif, le dispositif d'émission (4) est conçu pour émettre le signal utile (3) polarisé dans une première direction, le réflecteur de polarisation passif (8) est conçu pour tourner la polarisation du signal utile (3) dans une deuxième direction différente de la première direction et continuer à émettre le signal utile (3), et le dispositif de réception (5) est conçu pour recevoir de manière sélective dans la deuxième direction le signal utile (3) polarisé dans la deuxième direction.

**4.** Dispositif (1) selon la revendication 3, **caractérisé en ce que** le dispositif d'émission (4) et le dispositif de réception (5) sont configurés avec une antenne commune, notamment des trajets différents pour le dispositif d'émission (4) d'un côté et le dispositif de réception (5) de l'autre côté sont configurés dans un guide d'ondes de l'antenne, conformément aux directions de polarisation du signal utile (3) émis et reçu.

**5.** Dispositif (1) selon la revendication 1 ou 2, **caractérisé en ce que** le dispositif de polarisation (9) est un dispositif de polarisation actif, le dispositif d'émission (4) est conçu pour émettre le signal utile (3) polarisé dans une première direction, le dispositif de polarisation (9) actif est conçu pour tourner et émettre le signal utile (3) dans une deuxième direction, et le dispositif de réception (5) est conçu pour recevoir le signal utile (3) polarisé dans la deuxième direction et le dispositif d'interprétation (6) est conçu pour effectuer une interprétation vectorielle du signal total reçu, de sorte que le signal utile (3) polarisé dans la deuxième direction peut être différencié d'autres parties du signal total reçu.

**6.** Dispositif (1) selon la revendication 1 ou 2, **caractérisé en ce que** sont prévus un premier dispositif de polarisation (8) passif et un deuxième dispositif de polarisation (9) actif, le dispositif d'émission (4) et le dispositif de réception (5) présentent une première orientation de polarisation, le deuxième dispositif de polarisation (9) actif présente une deuxième orientation de polarisation différente de la première orientation de polarisation, le dispositif d'émission (4) est conçu pour polariser le signal utile (3) dans la première orientation de polarisation et pour l'émettre à travers le fluide (2), le premier dispositif de polarisation (8) passif est conçu pour tourner le signal utile (3) dans la deuxième orientation de polarisation et pour l'émettre à travers le fluide (2), le deuxième dispositif de polarisation (9) actif est conçu pour tourner le signal utile (3) et pour l'émettre à travers le fluide (2), le premier dispositif de polarisation (8) passif est conçu pour tourner le signal utile (3) dans la première orientation de polarisation et pour l'émettre à travers le fluide (2), le dispositif de réception (4) est conçu pour recevoir le signal utile (3) et le dispositif d'interprétation (6) est conçu pour effectuer une interprétation vectorielle du signal total reçu, de sorte que le signal utile (3) polarisé dans la deuxième direction peut être différencié d'autres parties du signal total reçu.

**7.** Dispositif (1) selon la revendication 6, **caractérisé en ce que** le dispositif d'émission (4), le dispositif de réception (5) et le deuxième dispositif de polarisation (9) actif sont configurés avec une antenne commune, le dispositif d'émission (4) et le dispositif de réception (5) présentent une première orientation de polarisation et le deuxième dispositif de polarisation (9) actif présente une deuxième orientation de polarisation différente de la première orientation de polarisation.

**8.** Dispositif (1) selon la revendication 7, **caractérisé en ce que** deux trajets différents pour le dispositif d'émission (4) et le dispositif de réception (5) d'un côté et pour le deuxième dispositif de polarisation (9) actif de l'autre côté sont notamment configurés dans un guide d'ondes de l'antenne commune, conformément aux orientations de polarisation du signal utile (3) émis par le dispositif d'émission (4) et reçu par le dispositif de réception (5) d'un côté et du signal utile (3) reçu et émis par le deuxième dispositif de polarisation (9) actif.

**9.** Dispositif selon l'une des revendications 5 à 8, **caractérisé en ce que** le dispositif de polarisation (9) actif est réalisé sous la forme d'un modulateur, notamment sous la forme d'un modulateur destiné à réaliser des coefficients de réflexion réglables, de préférence sur la base de commutateurs à haute fréquence.

**10.** Procédé pour faire fonctionner un dispositif (1) destiné à déterminer la part volumique d'au moins un composant d'un fluide (2) à plusieurs phases en se basant sur le temps de propagation d'un signal utile (3) électromagnétique émis dans le fluide (2) à plusieurs phases selon l'une des revendications 5 à 9, comprenant au moins un dispositif de polarisation (9) actif, une première influence de polarisation étant réglée sur le dispositif de polarisation (9) actif lors d'une première mesure et un premier signal reçu étant capté par le dispositif de réception (5), une deuxième influence de polarisation, différente de la première influence de polarisation, étant réglée sur le dispositif de polarisation (9) actif lors d'une deuxième mesure et un deuxième signal reçu étant capté par le dispositif de réception (5), et le premier signal reçu ainsi que le deuxième signal reçu étant superposés de telle sorte que les signaux parasites contenus dans le premier signal reçu et ceux contenus dans le deuxième signal reçu s'annulent, notamment qu'également les signaux utiles contenus dans le premier signal reçu et ceux contenus dans le deuxième signal reçu s'additionnent.

**11.** Procédé selon la revendication 10, **caractérisé en ce que** lors de la première mesure, un coefficient de réflexion positif est réglé sur le deuxième dispositif de polarisation (9) actif et lors de la deuxième mesure, un coefficient de réflexion négatif est réglé sur le deuxième dispositif de polarisation (9) actif, notamment le changement de phase du signal utile étant détecté lors de la deuxième mesure et son signe étant pris en compte.

(Stand der Technik)

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SIHVOLA, A.** Mixing Rules with complex Dielectric Coefficients. *Subsurface Sensing Technologies and Applications,* 2000, vol. 1 (4

- **BAER, C. ; MUSCH, T. ; GERDING, M.** Conceptual Design of a Procedure for Density Monitoring of Pulverized Fuels in Pneumatic Conveying Systems with Microwaves (8-12 GHz). *Proceedings of the 6th German Microwave Conference,* 14. Marz 2011